# EUROPEAN PATENT SPECIFICATION

(11) **EP 0 861 643 B1**
(45) Date of publication and mention of the grant of the patent: **24.10.2001**
(21) Application number: 98301373.1
(22) Date of filing: 25.02.1998
(51) Int. Cl.: A61F 13/15

(54) **Urine absorbent bag**
Urinabsorbierender Beutel
Sac absorbant l'urine

(30) Priority: 27.02.1997 JP 4355297
(43) Date of publication of application: 02.09.1998
(73) Proprietor: UNI-CHARM CORPORATION, Kawanoe-shi Ehime-ken (JP)
(72) Inventor: Nozaki, Satsohi, Imabari-shi, Ehime-ken (JP); Maeno, Takashi, Kawanoe-shi, Ehime-ken (JP); Utsunomiya, Makoto, Chiba-shi, Chiba-ken (JP); Sakamoto, Yutaka, Yokohama-shi, Kanagawa-ken (JP)
(74) Representative: Parry, Christopher Stephen

(56) References cited:
- WO-A-88/06008
- US-A- 4 559 051
- US-A- 4 627 846

## Description

This invention relates to urine absorbent bags for bedridden patients and persons suffering from incontinence.

A urine absorbent bag disclosed in Japanese Utility Model Application Publication (Kokoku) No Sho61-30653 comprises a pair of pads, each comprising a liquid-permeable topsheet, a liquid-impermeable backsheet and a liquid-absorbent core disposed between these two sheets, placed one upon another and sealed together along peripheral edges of these two pads so as to form a bag. One of these pads placed one upon another is formed with a through-hole for insertion of a wearer's penis.

With such urine absorbent bag of prior art, the wearer must force his penis in the bag from an outside thereof and therefore this often makes it difficult to wear the bag smoothly particularly when the wearer is aged patient. While the through-hole may be dimensioned to be relatively large in order to solve this problem, this inevitably increases any apprehension that the bag might fall off from the wear's penis after the bag has been worn.

US-A-4627846 discloses an incontinence shield for men which has a front-piece and a back-piece and a centre-piece which is narrower in relation thereto. The shield is folded double and sealed along three sides to form a pouch which is open at the top. The shield is arranged to embrace both the penis and the scrotum of the wearer and to this end the back-piece has a recess extending from the open end edge thereof.

In view of the problem as has been described above, it is a principal object of the invention to provide a urine absorbent bag improved to be easily worn and substantially free from any apprehension that the bag might fall off from the wearer's penis after the bag has been worn.

The object set forth above is achieved, according to the invention, by a urine absorbent bag formed by a pair of pad sections each comprising a liquid-permeable topsheet, a liquid-impermeable backsheet and a liquid-absorbent core disposed between these two sheets, wherein peripheral edges of the respective pad sections placed one upon another are partially sealed together and portions remaining at the peripheral edges are left non-sealed so as to form an opening and wherein one of the pad sections is formed with guide means extending therethrough for insertion of a wearer's penis; characterised in that the bag is formed in a substantially rectangular shape the peripheral edges of which comprise a pair of longitudinally opposite ends extending in parallel to each other transversely of the bag and a pair of transversely opposite side edges extending in parallel to each other longitudinally of the bag; the pad sections placed one upon another are sealed together along one of the longitudinally opposite ends and over an extent of the transversely opposite side edges extending continuously from the sealed one of the longitudinally opposite ends and are left non-sealed along the other of the longitudinally opposite ends and over the remaining extent of the side edges so that regions of the respective pad sections defined by the extent of the peripheral edges left non-sealed can be folded outwardly of the urine absorbent bag; and one of these foldable regions of the respective pad sections is formed, at a location intended to be exposed as the corresponding region of the other pad section is folded outwardly of the bag, with the guide means.

Fig 1 is a perspective view showing an embodiment of a urine absorbent bag according to the invention as partially broken away;

Fig 2 is a sectional view taken along a line II - II in Fig 1, in which upper and lower pads are placed one upon another.

Fig 3 is a sectional view taken along a line III - III in Fig 1, in which upper and lower pads are placed one upon another.

Fig 4 is a perspective view showing another embodiment of the invention;

Fig 5 is a sectional view taken along a line V - V in Fig 4; and

Fig 6 is a view similar to Fig 1 showing still another embodiment of the invention.

Details of a urine absorbent bag according to the invention will be more fully understood from the description given hereunder in reference with the accompanying drawings.

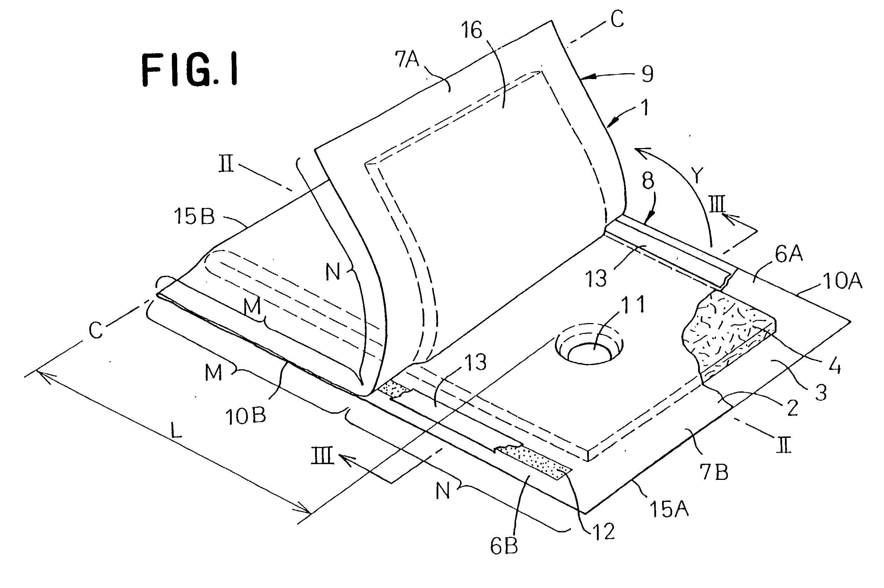

Figs. 1, 2 and 3 are respectively a perspective view showing a urine absorbent bag as partially broken away, a sectional view taken along a line II - II in Fig. 1 and a sectional view taken along a line III - III in Fig. 1. This specific embodiment of the urine absorbent bag is formed by folding a single rectangular pad 1 comprising a liquid-permeable topsheet 2, a liquid-impermeable backsheet 3 and a liquid-absorbent core 4. The topsheet 2 and the backsheet 3 are placed one upon another and bonded together along portions thereof extending outward beyond a peripheral edge of the absorbent core 4 by means of hot melt adhesive or heat-sealing effect of at least one of the sheets 2, 3 so as to form a pair of transversely opposite side edges 6A, 6B extending in parallel to each other longitudinally of the pad 1 and a pair of longitudinally opposite ends 7A, 7B extending in parallel to each other transversely of the pad 1. The pad 1 is folded with the topsheet 2 lying inside along a center line C - C extending across a full width of the pad 1 in longitudinally lower and upper pad sections 8, 9 which form together a rectangular urine absorbent bag. The urine absorbent bag formed in this manner has one end 15A defined by the pair of longitudinally opposite ends 7A, 7B, the other end 15B extending along the center line C - C in parallel to the one end 15A and a pair of transversely opposite side edges 10A, 10B defined by the side edges 6A, 6B, respectively, extending in parallel to each other.

The lower pad section 8 is formed in a transversely middle region thereof with guide means for insertion of a wearer's penis in the form of a circular hole extending through the pad section 8 in the direction of thickness thereof. The side edges 6A, 6B of the lower pad section 8 are bonded in a water-tight fashion to the side edges 6A, 6B of the upper pad section 9 over a first zone M of the side edges 6A, 6B longitudinally extending from the center line C - C, i.e., the urine absorbent bag is peripherally sealed in this first zone M. Over a second zone N of the side edges 6A, 6B extending between the first zone M and the end 7B, the pair of pads 8, 9 are not peripherally sealed together. Over the second zone N of the side edges 6A, 6B, however, the lower pad section 8 is applied with adhesive agents 12 by means of which the upper pad section 9 can be peripherally bonded to the lower pad section 8 and the adhesive agents 12 are protectively covered with release sheets before the bag is actually used.

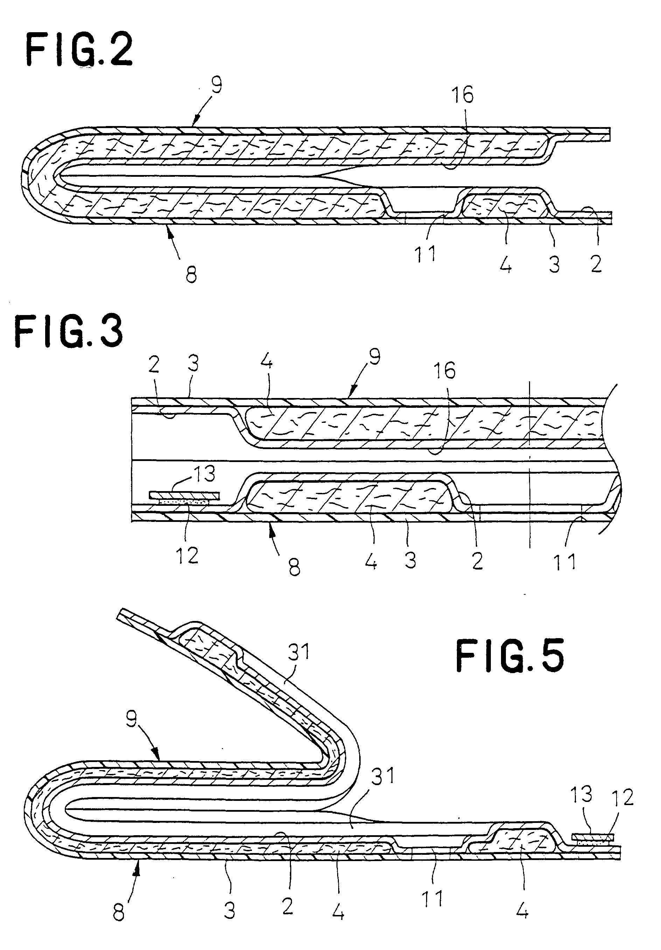

Regions of the respective pad sections 8, 9 extending between the second zones N of the side edges 6A, 6B, the pad sections 8, 9 placed one upon another can be folded outward. Regarding the upper pad section 9, a square U-shaped region 16 defined by the second zone N of the respective side edges 6A, 6B and the one end 7A can be folded outward of the bag as indicated by an arrow Y. Fig. 1 shows a state in which the region 16 has been folded outward of the bag and an inner side of the lower pad section 8 is partially exposed. A region of the lower pad section 8 thus exposed is formed with the guide means 11 for insertion of a wearer's penis. Figs. 2 and 3 show the bag as the region 16 of the upper pad section 9 has been placed upon the lower pad section 8.

A length of the first zone M of the respective side edges 6A, 6B in the lower and upper pad sections 8, 9 extending from the center line C - C toward the ends 7A, 7B is dimensioned to less than a linear distance L as measured from the center line C - C to a periphery of the guide means 11 for insertion of a wearer's penis in order to assure that the guide means 11 for the insertion is adequately exposed as the region 16 is folded outward of the bag as shown by Fig. 1.

With the urine absorbent bag constructed as has been described above, the wearer may insert his fingers into the guide means 11 from the inner side toward the outer side of the bag to guide his penis from the outer side into the inner side of the bag. The penis once having been guided into the bag may be laid so as to be oriented to the center line C - C or to the side edge 7A or 7B. In any case, after the penis has been laid, the release sheets 13 are peeled off from the adhesive agents 12 and then the region 16 of the upper pad section 9 is placed upon the lower pad section 8 so that these pad sections 8, 9 may be peripherally bonded together.

In the case of this embodiment of the urine absorbent bag, the first zones M of the respective side edges 6A, 6B may be reliably urine-tight-sealed together in the course of manufacturing and the penis may be laid so as to be oriented to the center line C - C to avoid any apprehension that urine leak might occur due to a defective closure of the bag by a helper or a wearer himself using the adhesive zones. This bag allows for a relatively high freedom of the fingers of a wearer for insertion of this penis. Therefore, fitting of peripheral edge of the guide means 11 around the penis can be improved by appropriately reducing the diameter of the guide means 11 in the form of the circular through-hole without making it significantly difficult to insert the penis into the through-hole. The guide means 11 for insertion of a wearer's penis may be provided along the peripheral edge thereof with an elastic member not only to facilitate insertion of the fingers into the guide means 11 but also to improve the fitting around the penis. In a consequence, both leakage of urine from the guide means 11 and falling off of the urine absorbent bag from the penis can be effectively avoided.

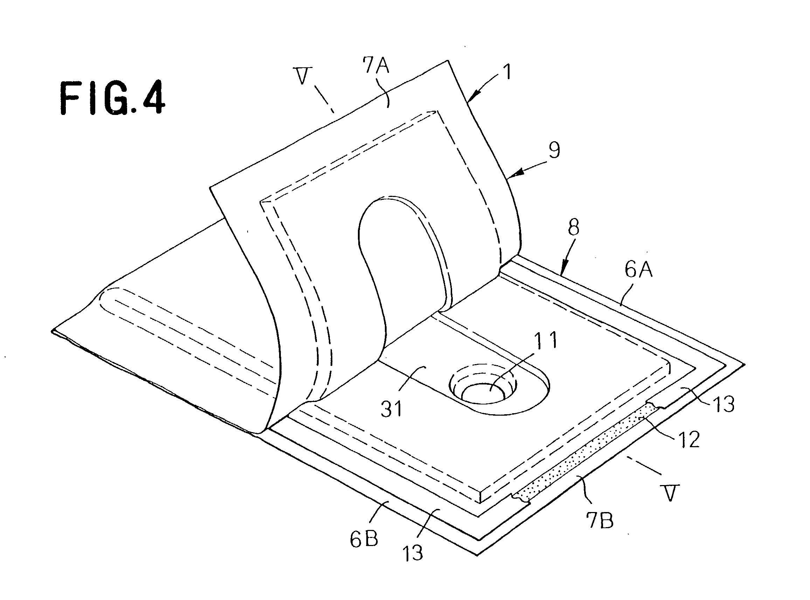

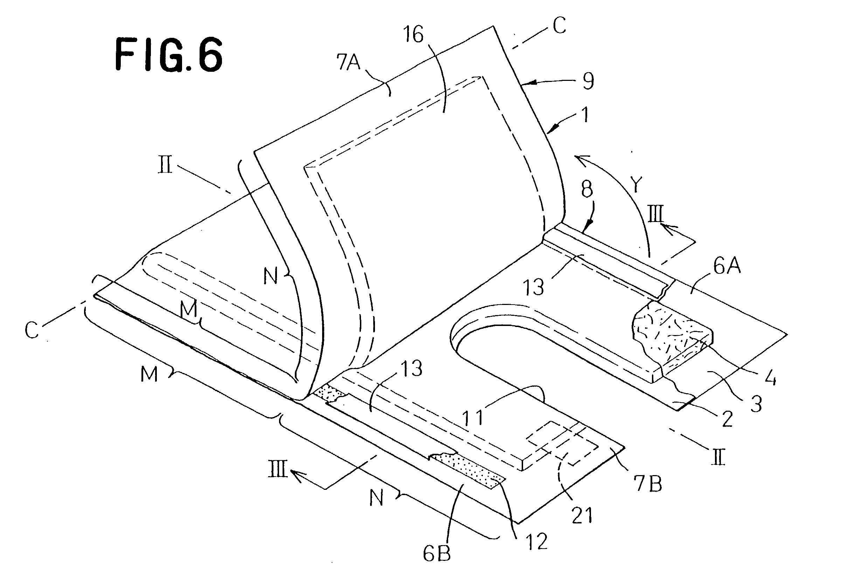

Figs. 4 and 5 are respectively a view similar to Fig. 1 and a sectional view taken along a line V - V in Fig. 4 both showing another embodiment of the urine absorbent bag according to the invention. According to this embodiment, the inner side of the urine absorbent bag is formed in a transversely middle region of the lower pad section 8 with a groove 31 extending in parallel to the side edges 6A, 6B. The groove 31 preferably extends also in the corresponding region of the upper pad section 9. The penis inserted into the bag may be laid within the groove 31 to stabilize position as well as orientation of the penis within the urine absorbent bag. By positioning the penis in the middle region of the bag in this manner, discharged urine more evenly and quickly spreads transversely of the absorbent core 4 and leakage of urine is effectively avoided than the case in which the penis is positioned to one side within the urine absorbent bag. According to this embodiment, the lower pad section 8 is applied along the side edges 6A, 6B and the one end 7B with the adhesive agent 12 so as to describe a square U-shape. The adhesive agent 12 is protectively covered with the release sheet 13 before the bag is actually used.

Fig. 6 is a view similar to Fig. 1 showing still another embodiment of the urine absorbent bag. According to this embodiment, the guide means 11 for insertion of a wearer's penis comprises a U-shaped cutout, instead of the circular through-hole, provided in the lower pad section 8. An outer surface of the backsheet 3 is formed in the proximity of the guide means 11 for insertion of a wearer's penis with a fastening zone 21 applied with an adhesive agent so that portions of the lower pad section 8 extending along the U-shaped cutout may be fastened around the proximal end of the penis in order to avoid falling off of the bag from the penis.

It should be understood that the urine absorbent bag may be formed by placing two separate pad members one upon another without departing from the scope of the invention. In this case, a thickness as well as composition of the liquid-absorbent core 4 in one of the pad members 8, 9 can be freely selected independently of the other pad member.

The urine absorbent bag according to the invention comprises the upper and lower pad sections placed one upon another, wherein one of these pad sections is formed with the guide means for insertion of a wearer's penis and the region of the other pad section covering the guide means is foldable outwardly of the bag. The guide means for a wearer's insertion of penis is exposed as the region is folded outwardly of the bag. With the wearer's fingers inserted into the guide means from the inner side toward the outer side of the bag, it is easily achieved to guide the penis into the bag.

The guide means for insertion of a wearer's penis in the form of the circular through-hole advantageously makes it easy to fit the peripheral edge of the guide means around the penis.

The guide means for insertion of a wearer's penis in the form of the U-shaped cutout advantageously facilitates insertion of the penis.

The transversely opposite side edges as well as the longitudinally opposite ends of the urine absorbent bag initially in non-sealed state may be applied with adhesive to assure that the bag can be urine-tightly closed after the penis has been inserted into the bag.

The groove formed inside the urine absorbent bag advantageously enables position and orientation of the penis within the bag to be stabilized.

The urine absorbent bag formed by folding a single pad in two advantageously simplifies manufacturing of the urine absorbent bag in comparison with the case in which a pair of separately prepared pads are placed one upon another to form the urine absorbent bag.

However, the urine absorbent bag formed by placing a pair of separately prepared pads is advantageous in that a thickness as well as a composition of the liquid-absorbent core in each of these two pads can be freely selected independently of the other pad.

## Claims

1. A urine absorbent bag formed by a pair of pad sections (8, 9) each comprising a liquid-permeable topsheet (2), a liquid-impermeable backsheet (3) and a liquid-absorbent core (4) disposed between these two sheets, wherein peripheral edges of the respective pad sections placed one upon another are partially sealed together and portions remaining at the peripheral edges are left non-sealed so as to form an opening and wherein one of the pad sections is formed with guide means (11) extending therethrough for insertion of a wearer's penis; **characterised in that** the bag is formed in a substantially rectangular shape the peripheral edges of which comprise a pair of longitudinally opposite ends (15A, 15B) extending in parallel to each other transversely of the bag and a pair of transversely opposite side edges (10A, 10B) extending in parallel to each other longitudinally of the bag; the pad sections (8, 9) placed one upon another are sealed together along one of the longitudinally opposite ends (15B) and over an extent (M) of the transversely opposite side edges (10A, 10B) extending continuously from the sealed one (15B) of the longitudinally opposite ends and are left non-sealed along the other (15A) of the longitudinally opposite ends and over the remaining extent (N) of the side edges (10A, 10B) so that regions (16) of the respective pad sections (8, 9) defined by the extent (N) of the peripheral edges left non-sealed can be folded outwardly of the urine absorbent bag; and one of these foldable regions of the respective pad sections is formed, at a location intended to be exposed as the corresponding region of the other pad section is folded outwardly of the bag, with the guide means (11).

2. A urine absorbent bag according to Claim 1, wherein the guide means for insertion of a wearer's penis is provided in the form of a circular through-hole (11).

3. A urine absorbent bag according to Claim 1, wherein the guide means for insertion of a wearer's penis is provided in the form of a substantially U-shaped cutout (11) formed in the pad section.

4. A urine absorbent bag according to Claim 2, wherein the extents (N) of the pair of transversely opposite side edges left non-sealed are at least partially applied with an adhesive agent (12) so that the non-sealed extent (N) of the respective side edges can be partially sealed by means of the adhesive agent after insertion of the penis.

5. A urine absorbent bag according to Claim 4, wherein the other end (15A) of the urine absorbent bag is also applied on an inner surface thereof with an adhesive agent (12).

6. A urine absorbent bag according to Claim 1, wherein, of the pair of pad sections (8, 9) placed one upon another, at least the pad section having the guide means (11) for insertion of a wearer's penis is formed on an inner side thereof in a middle between the pair of transversely opposite side edges and including the guide means for insertion of a wearer's penis with a groove (31) extending in parallel to the side edges.

7. A urine absorbent bag according to Claim 1, wherein the pair of pad sections (8, 9) placed one upon another are formed by folding a single rectangular pad along a center line (C-C) extending across a full width of the pad so as to divide the pad in longitudinally two sections.

8. A urine absorbent bag according to Claim 1, wherein the pair of pad sections placed one upon another comprise a pair of separately prepared pads placed one upon another.

## Patentansprüche

1. Urinabsorbierender Beutel, der aus einem Paar Vorlageteilen (8, 9) geformt ist, wobei jedes einen flüssigkeitsdurchlässigen Deckbogen (2), einen flüssigkeitsdurchlässigen rückwärtigen Bogen (3) und einen flüssigkeitsabsorbierenden Kern (4) umfasst, der zwischen den zwei Bogen angeordnet ist, worin die peripheren Kanten der betreffenden aufeinander gelegten Vorlageteile teilweise miteinander versiegelt sind und Abschnitte an den peripheren Kanten unversiegelt gelassen werden, um eine Öffnung zu formen und worin eines der Vorlageteile mit dem Führungsmittel (11) geformt ist, das sich durch diese, zwecks Einschieben des Penis der Trägerperson, erstreckt; **dadurch gekennzeichnet, dass** der Beutel im wesentlichen rechteckige Form aufweist, deren periphere Kanten ein Paar longitudinale gegenüberliegende Enden (15A, 15B), die sich parallel zueinander in Querrichtung des Beutels erstrecken und ein Paar sich transversal gegenüberliegende Enden (10A, 10B) umfassen, die sich parallel zueinander in Längsrichtung des Beutels erstrecken; die aufeinander gelegten Vorlageteile (8,9) werden entlang eines der longitudinal gegenüberliegenden Enden (15B) und über einen Bereich (M) der transversal gegenüberliegenden Kanten (10A, 10B), der sich kontinuierlich ab dem versiegelten Ende (15b) der longitudinal gegenüberliegenden Enden erstreckt, miteinander versiegelt und werden entlang dem anderen Ende (15A) der longitudinal gegenüberliegenden Enden und über den verbleibenden Bereich (N) der Seitenkanten (10A, 10B) unversiegelt gelassen, so dass Regionen (16) der betreffenden Vorlageteile (8,9), die durch den Bereich (N) der unversiegelt gelassenen peripheren Kanten definiert sind, vom urinabsorbierenden Beutel nach außen gefaltet werden können; und einer dieser faltbaren Bereiche der betreffenden Vorlageteile ist an einer Stelle gebildet, die absichtlich als der entsprechende Bereich des anderen Vorlageteils freigelegt werden soll, der mit den Führungsmitteln (11) vom Beutel nach außen gefaltet wird.

2. Urinabsorbierender Beutel nach Anspruch 1, worin das Führungsmittel für das Einschieben des Penis der Trägerperson in Form eines runden durchgehenden Lochs (11) bereitgestellt wird.

3. Urinabsorbierender Beutel nach Anspruch 1, worin das Führungsmittel für das Einschieben des Penis der Trägerperson in Form eines im wesentlichen U-förmigen Ausschnitts (11) bereitgestellt wird, der im Vorlageteil geformt ist.

4. Urinabsorbierender Beutel nach Anspruch 2, worin die Ausmaße (N) des Paares transversal gegenüberliegender Seitenkanten, die unversiegelt gelassen wurden, wenigstens teilweise mit einem Klebemittel (12) versehen sind, so dass der unversiegelte Bereich (N) der betreffenden Seitenkanten nach Einschieben des Penis teilweise mittels des Klebemittels versiegelt werden kann.

5. Unrinabsorbierender Beutel nach Anspruch 4, worin das andere Ende (15A) des urinabsorbierenden Beutels auch auf einer Innenfläche mit einem Klebemittel (12) versehen ist.

6. Urinabsorbierender Beutel nach Anspruch 1, worin vom Paar aufeinander gelegter Vorlageteile (8,9) wenigstens das Vorlageteil, welches das Führungsmittel (11) zum Einschieben des Penis der Trägerperson aufweist, auf einer Innenseite davon in einer Mitte zwischen dem Paar transversal gegenüberliegender Seitenkanten und einschließlich des Führungsmittels zum Einschieben des Penis der Trägerperson, mit einer Aussparung (31) geformt wird, die sich parallel zu den Seitenkanten erstreckt.

7. Urinabsorbierender Beutel nach Anspruch 1, worin das Paar aufeinander gelegter Vorlageteile (8,9) geformt wird, indem eineeinzelne rechteckige Vorlage entlang einer Mittellinie (C-C), die sich quer über eine volle Breite der Vorlage erstreckt, gefaltet wird, um die Vorlage longitudinal in zwei Teile zu unterteilen.

8. Urinabsorbierender Beutel nach Anspruch 1, worin das Paar aufeinander gelegter Vorlageteile ein Paar separat präparierter Vorlagen umfaßt, die aufeinander gelegt werden.

## Revendications

1. Poche absorbant l'urine, formée par une paire de sections de couche (8, 9) comprenant chacune une feuille de dessus perméable aux liquides (2), une feuille de dos imperméable aux liquides (3) et une partie centrale absorbant les liquides (4) disposée entre ces deux feuilles, dans laquelle les bords périphériques des sections de couche respectives placées l'une sur l'autre sont en partie scellées ensemble et les parties restantes au niveau des bords périphériques sont laissées non scellées de façon à former une ouverture, et dans laquelle l'une des sections de couche est munie d'un moyen de guidage (11) s'étendant au travers de celle-ci en vue de l'insertion du pénis de celui qui la porte, **caractérisée en ce que** la poche est réalisée suivant une forme sensiblement rectangulaire dont les bords périphériques comprennent une paire d'extrémités longitudinalement opposées (15A, 15B) s'étendant parallèlement l'une à l'autre dans le sens transversal de la poche, et une paire de bords latéraux transversalement opposés (10A, 10B) s'étendant parallèlement l'un à l'autre dans le sens longitudinal de la poche, les sections de couche (8, 9) placées l'une sur l'autre étant scellées ensemble le long de l'une des extrémités longitudinalement opposées (15B) et sur une étendue (M) des bords latéraux transversalement opposés (10A, 10B) s'étendant en continu depuis l'extrémité scellée (15B) des extrémités longitudinalement opposées, et étant laissées non scellées le long de l'autre (15A) des extrémités longitudinalement opposées et sur l'étendue restante (N) des bords latéraux (10A, 10B), de sorte que des régions (16) des sections de couche respectives (8, 9) définies par l'étendue (N) des bords périphériques laissés non scellés peuvent être repliées vers l'extérieur de la poche absorbant l'urine, et l'une de ces régions repliables des sections de couche respectives est munie, à un emplacement prévu pour être exposé lorsque la région correspondante de l'autre section de couche est repliée vers l'extérieur de la poche, du moyen de guidage (11).

2. Poche absorbant l'urine selon la revendication 1, dans laquelle le moyen de guidage destiné à l'insertion du pénis de celui qui la porte est prévu sous forme d'un orifice traversant circulaire (11).

3. Poche absorbant l'urine selon la revendication 1, dans laquelle le moyen de guidage destiné à l'insertion du pénis de celui qui la porte est prévu sous forme d'une découpe (11) sensiblement en forme de U formée dans la section de couche.

4. Poche absorbant l'urine selon la revendication 2, dans laquelle les étendues (N) de la paire de bords latéraux transversalement opposés laissés non scellés reçoivent au moins en partie l'application d'un agent adhésif (12), de telle sorte que l'étendue non scellée (N) des bords latéraux respectifs puisse être en partie scellée au moyen de l'agent adhésif après l'insertion du pénis.

5. Poche absorbant l'urine selon la revendication 4, dans laquelle l'autre extrémité (15A) de la poche absorbant l'urine reçoit également l'application d'un agent adhésif (12) sur une surface intérieure de celle-ci.

6. Poche absorbant l'urine selon la revendication 1, dans laquelle, parmi la paire des sections de couche (8, 9) placées l'une sur l'autre, au moins la section de couche comportant le moyen de guidage (11) destiné à l'insertion du pénis de celui qui la porte est munie, sur une face intérieure de celle-ci, au milieu entre la paire de bords latéraux transversalement opposés et comprenant le moyen de guidage destiné à l'insertion du pénis de celui qui la porte, d'une gouttière (31) s'étendant parallèlement aux bords latéraux.

7. Poche absorbant l'urine selon la revendication 1, dans laquelle la paire de sections de couche (8, 9) placées l'une sur l'autre est formée en repliant une simple couche rectangulaire suivant une ligne centrale (C-C) s'étendant au travers d'une largeur totale de la couche, de façon à diviser la couche en deux sections dans le sens longitudinal.

8. Poche absorbant l'urine selon la revendication 1, dans laquelle la paire de sections de couche placées l'une sur l'autre comprend une paire de couches préparées de façon séparée placées l'une sur l'autre.
